# EUROPEAN PATENT APPLICATION

(11) **EP 4 461 250 A1**
(43) Date of publication of application: **13.11.2024**
(21) Application number: 23315199.2
(22) Date of filing: 12.05.2023
(51) Int. Cl.: A61B 34/20, A61B 90/30, A61B 90/00, A61B 34/10, A61B 17/15

(54) **A SURGICAL ROBOTIC SYSTEM FOR EXECUTING A BONE IMPLANT SURGICAL PLAN**

(71) Applicant: Ecential Robotics, 38610 Gieres (FR)
(72) Inventor: Thiebaut, Lucie, 38610 Gieres (FR); Leitner, François, 38610 Gieres (FR)
(74) Representative: INNOV-GROUP

(57) **Abstract**

The invention relates to a surgical robotic system for executing a bone implant surgical plan, comprising:
- a surgical robot comprising a first robotic arm (100) for manipulating a surgical tool during a surgical procedure at a surgical site;
- a control unit, comprising a processor programmed to direct the surgical robot to position the surgical tool,
wherein said processor program includes instructions for executing:
- defining the location of the surgical tool with respect to the surgical site.

It is essentially characterized in that:
- said processor program includes instructions for further executing the following steps:
- moving the surgical tool to a bone located within the surgical site, said location being predefined within the surgical plan; and
- marking said bone with the surgical tool, said marking corresponding to the position of a bone implant to be implanted in the bone according to the surgical plan.

## Description

The present invention relates to a surgical robotic system for executing a bone implant surgical plan.

A surgical robotic system is used for assisting a user (e.g. a surgeon) during a surgical intervention.

For example, in bone surgery, the user may have to implant one or several screws, or a bone implant, into a bone, e.g. a vertebra, a hip, a knee etc.

In this regard, the use of a localization system that can localize trackers in real-time may be used to carry out such manipulation, either in assistance of the surgeon or autonomously. Both the anatomical structure and the surgical tool can be localized, which allows determining in real time the relative positions of the surgical tool relative to the anatomical structure to be treated.

To that end, both the surgical tool and the anatomical structure may comprise a tracker rigidly attached thereto, each tracker being tracked by the localization system, therefore also called tracking system.

In some circumstances, a tracker may be rigidly attached to a part of the robotic system and not the surgical tool directly, allowing indirect localization of the tool based on knowledge at any time of the kinematic model of the robotic system between the tracker and the tool.

The surgical robotic system comprises a surgical robotic arm, having at least six degrees of freedom, and comprising an end effector generally holding a surgical tool. The surgical tool is to be placed with a given position and orientation relative to a surgical target. Once the surgical tool is placed relative to the surgical target, the surgical robotic system assists the surgeon in moving the surgical tool relatively to the surgical target. Thus, the surgeon may manoeuvre the surgical tool with the right position and orientation relative to the surgical target during the surgery, according to a surgical plan.

During a surgery such as a bone implant, the bone implant must be implanted with a very precise position relative to the bone.

To this end, it is known to implement ink pads, which are used manually to mark the bone where the bone implant is to be implanted. It is also known to use tracked stencil, which are manually adjusted on site to match the bone implant planned position, manually pinned down to the adjusted location, and manually used as a guide for a manual tool dedicated to imprint the bone implant negative into the bone.

These manual operations may lead to some imprecision of the relative position of the bone implant relatively to the bone. The present invention aims to reduce or suppress this drawback.

To this end, the present invention relates to a surgical robotic system for executing a bone implant surgical plan, said system comprising:
- a surgical robot comprising a first robotic arm having at least six degrees of freedom for manipulating a surgical tool within a surgical site during a surgical procedure for implementing said surgical plan;
- a control unit, comprising a processor programmed to direct the surgical robot to position the surgical tool at the surgical site,
wherein said processor program includes instructions for executing the following step:
o defining the location of the surgical tool with respect to the location of the surgical site, within a same three-dimensional coordinates system, in real-time.

It is essentially characterized in that:
- said processor program includes instructions for further executing the following steps:
   o moving the surgical tool to a bone located within the surgical site, said location being predefined within the surgical plan; and
   o marking said bone with the surgical tool, said marking corresponding to the position of a bone implant to be implanted in the bone according to the surgical plan.

Advantageously, the surgical robotic system according to the present invention relates can fully execute a bone implant surgical planning, meaning without manual operation of a surgeon directly on a patient's body.

The position of a bone implant to be implanted in the bone can also be understood as the position of a planned bone implant anchoring system or keyed connection to be implanted in the bone.

It may be provided that the processor program includes instructions for moving the surgical tool within the surgical site according to the surgical plan or to the surgical implant planned position.

It may be provided that the surgical tool is a passive surgical tool. For instance, it may be provided that the surgical tool is a stencil, which is inked with a biocompatible ink.

It may be provided that the surgical tool is motorized or not. For instance, it may be provided that the motorized tool is an electrical surgical tool or a pneumatical surgical tool, and for instance one amongst: an oscillating saw, a reciprocating saw, a Tuke saw, a drill.

For instance, it may be provided that the non-motorized tool is a chisel.

It may be provided that the surgical tool is an electrical scalpel.

Accordingly, the surgical tool may be understood as a cutting tool or a machining tool and indistinctively called "resecting tool". Similarly, "cutting a bone" and "resecting a bone" shall be understood as indistinct expressions.

It may be provided that said processor program includes instructions for executing the following step: resecting the bone located within the surgical site with the surgical tool, said surgical tool being a resecting tool, such that marking the bone and resecting the bone can be implemented with the same surgical tool.

Resecting a bone creates a resection area on said bone.

It may be provided that said processor program includes instructions for resecting the bone located within the surgical site with the surgical tool such that said resected bone presents a resection area, such that said bone can support a bone implant.

The resection area, or cut area, may be a flat area,
It may be provided that the instructions for executing the step of marking said bone that shall support the bone implant with the surgical tool, further comprise instructions for executing, at the surgical site, at least on one of the following steps:
a. Marking the bone that has been cut and that shall support the bone implant, on the cut area,
b. Marking the bone that has been cut and that shall support the bone implant away from the cut area.

It may be provided that the instructions for marking said bone with the surgical tool comprise marking on the bone the position, and in particular the central position, of a bone implant to be implanted in the bone, enabling keying in translation of the position of the implant.

It may be provided that the instructions for marking said bone with the surgical tool comprise marking on the bone the angular position of said bone implant, enabling keying in rotation of the position of the implant.

It may be provided that said bone implant comprises a set of at least two fins, the instructions for marking said bone with the surgical tool comprise marking on the bone the angular position of each fin of the set of fins.

It may be provided that the surgical robot further comprises a second robotic arm having at least six degrees of freedom for manipulating a surgical tool within a surgical site during a surgical procedure for implementing said surgical plan.

It may be provided that said control unit is located within said surgical robot.

It may be provided that the surgical site comprises a bone to receive the bone implant, said system further comprising a set of markers located on both said robotic arm and the bone to receive the bone implant; and a tracking system configured to locate the relative position of the robotic arm and the surgical site.

Preferably, markers are electromagnetic markers or optical markers, which can be identified by the localization system, which is further described.

Thanks to the present invention, the surgeon can perfectly execute his planning which, without the present invention, contains a purely manual step that does not guaranty the placement of the implant according to the surgical plan.

In the long term, the present invention allows the surgeon to draw conclusions as to which planning leads to the best clinical outcome in which clinical indication. This type of analysis is impossible to date as the real positioning of the implant with respect to its planned positioning remains unknown.

With the present invention, surgeons can collect data, and find correlations leading to the best clinical outcome. Thus, surgeons can design efficient patient specific clinical course of actions to reach the best possible outcome for each patient.

When the present invention is embodied as a bone marking system leaving an engraved mark in the bone and not just a print, the mark (or marks) serves (respectively serve) as a further risk control measure to ensure the correct execution of the implant placement. Indeed, as such, the mark (or marks) in the bone drives (respectively drive) the positioning of the implant print not only visually but physically as well, enabling keying in translation and/or in rotation of the position of the implant.

Other features and advantages of the present invention will appear in the detailed description that is given as a mere illustrative and non-limitative example.

### DRAWINGS

- Figure 1 illustrates a robotic arm according to the present invention, equipped with a set of trackers, in this case optical trackers, secured to a surgical tool;
- Figure 2 illustrates a robotic arm according to the present invention, mounted on a movable cart;
- Figure 3 illustrates a tibia and a femur both resected and marked with a surgical robotic system according to the present invention.

Surgical robotic systems are frequently used during surgical procedures to provide physicians with image-based information about a patient's anatomical situation and/or the position and orientation of a surgical instrument with respect to the patient.

Over the past decades, 2D images and three-dimensional (3D) imaging techniques have become more and more implemented.

Typically, an imaging system, e.g. such as an X-ray imaging system, moves about a given trajectory (e.g. a simple rotation about an axis or a more complex trajectory) in order to obtain images from different projection angles. A 3D volume of a body region of interest is reconstructed from said images.

Other techniques such as "Bone Morphing" are known to construct a 3D mesh of a body region of interest.

Such body region of interest comprises or is a surgical site, which is defined as being a 3D volume where a surgical plan shall be implemented, and within which movements of a surgical tool are preferably constrained.

### Surgical robotic system

A surgical robotic system according to the present invention, may comprise:
- a base, which may be a movable cart 200, or may be attached to an operating table,
- a robotic arm 100 having a proximal end 130 extending from a base, and a distal end 140 opposite to the proximal end 130, the distal end 140 comprising a surgical tool,
- a control unit, comprising a processor programmed to direct the surgical robot to position the surgical tool at the surgical site, the controller being configured to controllably move the robotic arm 100 according to a planned trajectory of a surgical plan, to implement said surgical plan during a surgical procedure.

The robotic arm 100 comprises a plurality of degrees of freedom in translation and/or rotation. Usually, the robotic arm 100 comprises at least five, and preferably six or seven motorized degrees of freedom. To that end, the robotic arm 100 comprises a plurality of articulated segments 110 driven by motors. By convention, the segments 110 are numbered from the proximal end 130 (which is the end closest to the movable cart 200) to the distal end 140 (opposite the proximal end 130) of the robotic arm 100. The successive robotic arm 100 joints may be rotations or translations. Successive rotations may be orthogonal or parallel. Some parts of the robotic arm 100 may also use parallel mechanisms such as a hexapod architecture.

The robotic system is active in the sense that it can hold and move a powered surgical tool that interacts directly with the anatomical structure, contrary to a passive robotic system that holds a guide in a predefined position with respect to the anatomical structure into which a powered surgical tool is inserted by a surgeon.

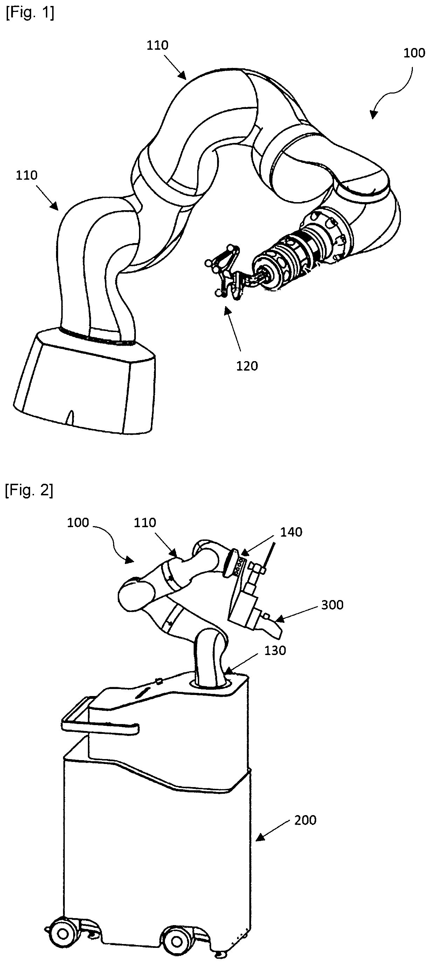

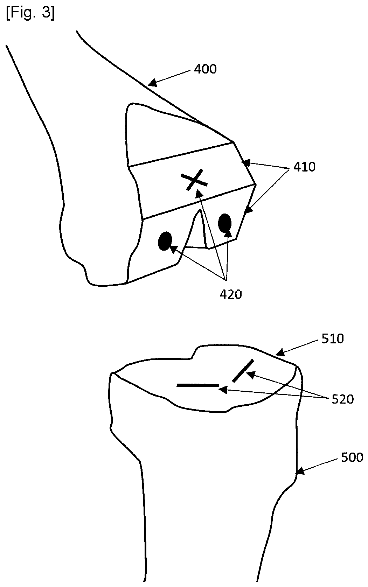

For example, a surgical saw is mounted on the robotic arm 100 end effector, as illustrated on figure 2 and actively marks, makes a notch or cuts the bone along a predefined path until an endpoint of a selected linear trajectory is reached, as illustrated on figure 3.

The robotic arm 100 is carried by the movable cart 200.

During a surgical operation, the cart 200 is intended to remain fixed relative to an operating table on which a patient lies, while the robotic arm 100 is moved to reach surgical target(s) within the surgical site according to a surgical plan.

The robotic arm 100 comprises a plurality of degrees of freedom in translation and/or rotation.

The robotic arm 100 may be either controlled in an autonomous mode according to desired targets and trajectories, or manipulated using a collaborative mode (cobot), or telemanipulated using a master control device. A combination of two or more of these different modes may be used on the same robotic system.

The distal end 140 of the robotic arm 100 holds a surgical tool.

In one embodiment, the surgical tool is a passive surgical tool. For instance, the surgical tool is a stencil, which is inked with a biocompatible ink.

In another embodiment, the surgical tool is a motorized surgical tool. For instance, the surgical tool is one amongst: an oscillating saw, a reciprocating saw, a Tuke saw, an electrical scalpel, a drill.

The surgical robotic system also includes a control unit configured to controllably move the robotic arm 100.

The control unit comprises a processor, a data storage device and a communication device. The control unit may advantageously be embedded in the movable cart 200.

In other embodiments, the control unit may be provided separate from the movable cart 200 and may be configured to communicate wirelessly or by wires with the robotic arm 100.

### Localization system

To define the location of the surgical tool with respect to the location of the surgical site, within a same three-dimensional coordinates system, in real-time, a localization system may be provided, which is also indistinctively called "tracking system".

Typically, as known in itself, the localization system is configured to localize trackers 120, or indistinctively markers, which can be electromagnetic or optical markers, attached to the patient, to the robotic arm 100, and/or the surgical tool as illustrated on figure 1.

Optical markers can be passive optical markers, with a reflecting surface, or active optical markers, with a LED.

The localization system can localize position and orientation of any tracker 120 mounted rigidly on the bones at the surgical site and on the surgical tool of the robotic system.

Said localization system can be an optical system, or an electromagnetic system, or any combination of optical, electromagnetic, ultrasonic, inertia measurement devices and sensors, or passive electro-mechanical arms with encoders.

For instance, the localization system comprises a camera arranged to detect optical trackers 120, wherein each optical tracker 120 comprises a set of reflective markers, having for example a spherical shape.

Typically, a first set of at least one tracker 120 is rigidly attached to a bone within or adjacent to the surgical site. A second set of at least one tracker 120 is rigidly attached to the surgical tool manipulated by the robotic arm 100.

The position and the orientation of the surgical tool is known in a coordinate system attached to the second set of at least one tracker 120 at any time. Preferably, the position and the orientation of the surgical tool is also known in a coordinate system attached to the first set of at least one tracker 120 at any time.

It is also possible to implement markerless solutions, such as shape recognition system using 3D scanners.

### Utilization of the robotic system

The robotic system may be operated as follows.

At the beginning of surgery, a patient is equipped with a first tracker 120 (called "patient tracker") detectable by the localization system.

A 3D image can be acquired either at the beginning of surgery using an X-ray imaging system itself (CBCT) or prior to surgery with another imaging system (CT or CBCT) and said 3D image is registered with respect to the patient tracker 120. Further techniques may be implemented for imagining the patient's surgical site, for instance bone morphing, 3D laser scanner or computer vision imagery with a stereo camera.

A plurality of trajectories of the surgical tool are planned in the 3D image. For that purpose, a surgical planning software is used to define surgical targets interactively or automatically in the 3D image.

The robotic system, which may be mobile on the wheels of the cart 200 forming the base of the robotic arm 100, is brought near the surgical table, as illustrated on figure 2 where the surgical robotic system comprises a surgical tool 300.

The robotic system is equipped with a surgical tool, as previously described.

A second tracker 120 (called "robot tracker") is mounted on the robotic arm 100, on the surgical tool directly or on any sub-system of the robotic system. The calibration of the robot tracker 120 with respect to the surgical tool can use several known calibration methods.

In a preferred embodiment, the position of the robot tracker 120 on the surgical tool is reproducible and always the same, and a localized pointer is used to check that a particular point of the surgical tool has precisely the expected coordinates with respect to the robot tracker 120.

In another preferred embodiment, the localized pointer is used to digitize at least three precisely defined points on the surgical tool and a point-based calibration is applied.

The robotic system may be moved manually (cobot) or automatically, to align the surgical tool to the planned trajectory, until it reaches an entry point on the bone surface at the surgical site.

Accordingly, the surgical tool is moved to a bone located within the surgical site, said location being predefined within the surgical plan.

The robotic system can be servo-controlled on the movements of the patient tracker 120 to maintain alignment with the entry point position on the bone, compensating for any motions of the bone due to patient's breathing or any mechanical interactions.

Once the surgical tool is located at the surgical site, the robotic arm 100 is controlled such that the surgical tool is moved with respect to the bone in order to prepare the surgery according to the surgical plan.

In this case, the surgery is a bone implant. In this kind of surgery, the position of the bone implant relative to the position of the bone is decisive for the patient outcome.

According to the present invention, the surgical tool is used, to mark said bone with the surgical tool, said marking corresponding to the position of a bone implant to be implanted in the bone according to the surgical plan.

For instance, in one embodiment, a cross can be marked on the bone to indicate the localization of the central position of a bone implant to be implanted in the bone.

In another embodiment, which is combinable with the previous embodiment, the surgical tool is used to mark on the bone the angular position of the bone implant to be implanted in the bone. For instance, the bone implant comprises a set of one or more fins and the surgical tool is used to mark the angular position of each fin of the set of fins on the bone.

A surgical plan may require that the bone within the surgical site be resect.

In this case, advantageously, marking the bone and resecting the bone can be implemented with the same surgical tool, saving a lot of time as there is no need to replace a surgical tool with another one.

When a bone within the surgical site is cut, the resecting area is a cutting area that may present a flat area. The bone may be partially or totally cut.

Thanks to the present invention, the flat area of the bone once cut may be marked, as Illustrated on figure 3.

On figure 3, a femur 400 has been cut. Accordingly, the femur 400 presents a resection area 410, in this case two resection areas 410, each of which being flat.

Thanks to the present invention, is it possible to implement a surgical tool to mark the bone. Accordingly, the resection area 410 of the femur comprise a set of at least on mark 420, for implanting a femoral component bone implant. Advantageously, the shape of the mark 420 may be chosen by the surgeon, e.g. a cross, a line or a dot. Advantageously, the chosen shape of the mark 420 will ease the implant of the bone implant.

For instance, on figure 3, the tibia 500 comprises a resection area 510 which comprises two marks 520 presenting the shape of a line. Each line corresponds to the position of a respective fin of a bone implant to be implanted, enabling keying in rotation of the position of the implant.

But also, the bone that has been cut may be marked away from the flat area, depending on the surgical plan or on the bone implant to be implanted.

The resection area may be partly flat. Typically, the shape of the resection area is chosen based on the surgical operation to be implanted.

Advantageously according to the present invention, marking the bone is simpler than machining the bone, as machining the bone requires a complete knowledge of the 3D shape of the bone implant.

It may be provided that the surgical robotic system comprises two robotic arms 100. Preferably the two robotic arms 100 are identical.

For instance, a first robotic arm 100 is equipped with a first surgical tool and a second robotic arm 100 is equipped with a second surgical tool. The second surgical tool may differ from the first surgical tool.

The first surgical tool can be used for marking the bone as previously described and the second surgical tool can be used for the surgery.

The present invention may be implemented for bone implants such as: knee, hips, shoulder, ankle, and elbow implants.

### Nomenclature

100 robotic arm
110 segment
120 tracker
130 proximal end
140 distal end
200 cart
300 surgical tool
400 femur
410 resection area of the femur
420 mark on the femur, for implanting a femoral component bone implant
500 tibia
510 resection area of the tibia
520 mark on the tibia, for implanting a tibial component bone implant

## Claims

1. A surgical robotic system for executing a bone implant surgical plan, said system comprising:
- a surgical robot comprising a first robotic arm (100) having at least six degrees of freedom for manipulating a surgical tool within a surgical site during a surgical procedure for implementing said surgical plan;
- a control unit, comprising a processor programmed to direct the surgical robot to position the surgical tool at the surgical site,
wherein said processor program includes instructions for executing the following step:
∘ defining the location of the surgical tool with respect to the location of the surgical site, within a same three-dimensional coordinates system, in real-time;
**Characterized in that**:
- said processor program includes instructions for further executing the following steps:
∘ moving the surgical tool to a bone located within the surgical site, said location being predefined within the surgical plan; and
∘ marking said bone with the surgical tool, said marking corresponding to the position of a bone implant to be implanted in the bone according to the surgical plan.

2. A surgical robotic system according to claim 1, wherein the surgical tool is a passive surgical tool.

3. A surgical robotic system according to claim 2, wherein the surgical tool is a stencil, which is inked with a biocompatible ink.

4. A surgical robotic system according to claim 1, wherein the surgical tool is a motorized surgical tool.

5. A surgical robotic system according to claim 4, wherein the surgical tool is an electrical surgical tool such as a cutting tool or a machining tool, and for instance one amongst: an oscillating saw, a reciprocating saw, a Tuke saw, a drill, an electrical scalpel.

6. A surgical robotic system according to claim 5, wherein said processor program includes instructions for executing the following step: cutting the bone located within the surgical site with the surgical tool, said surgical tool being a cutting tool, such that marking the bone and cutting the bone can be implemented with the same surgical tool.

7. A surgical robotic system according to claim 6, wherein said processor program includes instructions for cutting the bone located within the surgical site with the surgical tool such that said bone can support a bone implant.

8. A surgical robotic system according to claim 6 or 7, wherein the instructions for executing the step of marking said bone with the surgical tool, further comprise instructions for executing, at the surgical site, at least on one of the following step:
a. Marking the bone that has been cut and that shall support the bone implant, on the cut area,
b. Marking the bone that has been cut and that shall support the bone implant, away from the cut area.

9. A surgical robotic system according to any of the preceding claims, wherein the instructions for marking said bone with the surgical tool comprise marking on the bone the position of a bone implant to be implanted in the bone, in particular its central position, enabling keying in translation of the position of the implant.

10. A surgical robotic system according to any of the preceding claims, wherein the instructions for marking said bone with the surgical tool comprise marking on the bone the angular position of said bone implant, enabling keying in rotation of the position of the implant.

11. A surgical robotic system according to claim 10, wherein said bone implant comprises a set of at least two fins, the instructions for marking said bone with the surgical tool comprise marking on the bone the angular position of each fin of the set of fins.

12. A surgical robotic system according to any of the preceding claims, wherein the surgical robot further comprises a second robotic arm (100) having at least six degrees of freedom for manipulating a surgical tool within a surgical site during a surgical procedure for implementing said surgical plan.

13. A surgical robotic system according to any of the preceding claims, wherein said control unit is located within said surgical robot.

14. A surgical robotic system according to any of the preceding claims, wherein the surgical site comprises a bone to receive the bone implant, said system further comprising a set of markers located on both said robotic arm (100) and the bone to receive the bone implant; and a tracking system configured to locate the relative position of the robotic arm (100) and the bone at the surgical site.
